# EUROPEAN PATENT APPLICATION

(11) **EP 0 857 961 A2**
(43) Date of publication of application: **12.08.1998**
(21) Application number: 97306261.5
(22) Date of filing: 18.08.1997
(51) Int. Cl.: G01N 1/28

(54) **Sample preparing apparatus used with preparation disk with filter**

(30) Priority: 05.02.1997 JP 22861/97
(71) Applicant: MUTO PURE CHEMICALS COMPANY LTD., Tokyo (JP)
(72) Inventor: Muto, Yunosuka, Bunkyo-ku, Tokyo (JP)
(74) Representative: Pluckrose, Anthony William

(57) **Abstract**

The invention provides a sample preparing apparatus (10) used with a preparation disk (1) having a disk body (2) and an integral filter (3). The sample preparing apparatus (10) comprises a sample preparing section (21) which includes a disk support plate (9) having a holder (8) for holding the preparation disk (1) on the upper surface of the disk support plate (9), and a specimen guide (17) secured to the underside of the disk support plate (9). The specimen guide (17) has a suction port (13) connected to an evacuator for discharging specimen passed through the filter (3) of the prepartion disk (1), and a discharging section (23) coupled to the same preparing section.

## Description

This invention relates to a sample preparing apparatus used with a preparation disk having a filter.

Conventionally, a smear is prepared from cells or the like extracted from organisms and is used for cell examination to cytologically diagnose a lesion, and particularly this type of cell examination is broadly practised to diagnose cancer.

For example, in the examination of a urinary organ cell, a centrifugal precipitate coating method has been used as the most common method of preparing a smear. This method is inexpensive and simple in operation. However, it has disadvantages such as the fact that the centrifugal separation process is time-consuming in processing a large number of specimens, and it is not easy to uniformly coat the precipitate on a preparation disk with a pipette or a slide glass.

Under these circumstances, the applicant has developed a liquid specimen cell coating device which can treat a specimen with ease and inexpensively without centrifuging (Japanese Patent Application No. Hei 6-196586).

However, it was found that this device poses a sanitary problem because a specimen is discharged during a reverse operation, so that it may splash out, contaminating hands and nearby objects.

Therefore, the applicant newly proposed a preparation disk having a filter and a sample preparing apparatus used with the preparation disk and a method of using the preparation disk (Japanese Patent Application No. Hei 7-220003). The prior art preparation disk comprises a preparation disk body and an integral filter. The sample preparing apparatus comprises; as an upper part a waste liquid part which has an insertion section into which a preparation disk is inserted; as a lower part a waste liquid storage section; and a specimen storage section which can be detachably mounted above the waste liquid part in alignment with the preparation disk once inserted. Specifically, the preparation disk is mounted above the waste liquid part, the specimen storage section is fitted, a specimen contained in a specimen container such as a paper cup is poured into the specimen storage section, the air in the waste liquid part is sucked into the waste liquid storage section, and the specimen is filtered through a filter to prepare a sample.

However, if the specimen storage section is used once it has a liquid residue therein in an adhered state, and when another specimen is poured into the specimen storage section, the liquid residue may be mixed with the added specimen. To prevent mixing from taking place, the specimen storage section can be removed from the waste liquid section every time the specimen is examined, and replaced with a new specimen storage section. However, replacing is time-consuming and poor in efficiency. Also, a disposable specimen storage section is a heavy burden economically.

To achieve the above object, the sample preparing apparatus used with a preparation disk having a filter according to the invention uses a preparation disk which comprises a preparation disk body and an integral filter, and the apparatus comprises a sample preparing section, which includes a disk support plate having a holder for releasably holding the preparation disk on the upper surface of the disk support plate, and a specimen guide for discharging a specimen that has passed through the filter of the preparation disk which is secured to the underside of the disk support plate and which has a suction port connected to an evacuator; a suction member set on the disk support plate to couple a specimen container containing the specimen therein with the filter of the preparation disk; and a discharging section coupled to the sample preparing section.

The invention provides a sample preparing apparatus used with a preparation disk having a filter which does not require a lot of time to replace containers nor need a specimen storage section, prevents the liquid residue from being mixed, and does not become a heavy burden economically.

The liquid residue can thus be prevented from being mixed because the specimen container and the suction member are disposable, and the container can be replaced easily. Since only the specimen container and the suction member are disposable, an economical burden can be lowered.

Further, the suction member used for the sample preparing apparatus has a ring-like shape and has one end having the same inner diameter as the outer diameter of the filter of the preparation disk, and the other end having the same outer diameter as the outer diameter of the bottom of the specimen container. Thus, the specimen can be prevented from leaking between the specimen container and the preparation disk having a filter, and the preparation disk can be used effectively.

The suction member used for the sample preparing apparatus may have a large-diameter portion with one end thereof having substantially the same inner diameter as the diameter of the filter of the preparation disk having a filter, and a pipe-like suction section extending from the other end of the large-diameter portion. Thus, the specimen can be sucked up from above the specimen container, and operations can be further simplified because it is not necessary to form any hole in the bottom of the specimen container.

Also, in the sample preparing apparatus which uses the ring-like suction member the sample preparing section can further include container guide means secured to the upper surface of the support plate and for supporting the suction member and the specimen container above the preparation disk. Thus, the specimen container and the ring-like suction member can be mounted or removed easily, the liquid residue can be prevented from being mixed, and the specimen can be treated quickly and economically.

The sample preparing apparatus having the pipe-like suction member may have a sample preparing section further including suction member support means secured to the disk support plate and for supporting the suction member so as to be on the upper surface of the preparation disk. Thus, the suction member having the pipe-like suction can be easily mounted and secured to the disk support plate.

The sample preparing section preferably further includes a shield disposed inside the supporting pipe for shielding specimens falling through the specimen guide from the suction port in order to ensure that the specimen passing through the filter in the preparation disk having a filter is discharged into the discharging section. Also, a bypass duct for processing the liquid residue can be disposed so as to extend from a peripheral wall of the specimen guide, thereby facilitating the processing of the liquid residue in the specimen containing having a hole in its bottom.

Further, in the apparatus which has the ring-like suction member, the preparation disk, the suction member and the specimen container can be placed on the disk support plate in the sample preparing section and then a hole formed in the bottom of the specimen container. Specimen is sucked from below the disk support plate by means of an evacuator and filtered through the filter. Thus, a sample can be prepared efficiently.

In addition, the preparation disk and the suction member having pipe-like suction section can be placed on the disk support plate in the sample preparing section and the leading end of the sucking portion of the suction member inserted into the specimen container, with the specimen sucked from below the disk support plate by means of an evacuator and filtered through the filter. Thus, a sample can be prepared.

Preferred embodiments of the present invention will now be described with reference to the accompanying drawings, in which:
Fig. 1 is a perspective view showing an embodiment of the preparation disk having a filter used in the present invention;
Fig. 2 is a front view showing an embodiment of the sample preparing apparatus used with the preparation disk having a filter according to the invention;
Fig. 3 is a fragmentary sectional view of the embodiment of sample preparing apparatus shown in Fig. 2;
Fig. 4 is a fragmentary sectional view showing a different embodiment of the sample preparing apparatus used with the preparation disk having a filter according to the invention;
Fig. 5 is a fragmentary sectional view showing a further embodiment of the sample preparing apparatus used with the preparation disk having a filter according to the invention;
Fig. 6 is a front view showing a further embodiment of the sample preparing apparatus used with the preparation disk having a filter according to the invention;
Fig. 7 is a perspective view showing yet a further embodiment of the sample preparing apparatus according to the invention; and
Fig. 8 is a fragmentary perspective view showing still another embodiment of the sample preparing apparatus using a hanging-bell-shaped suction member according to the invention.

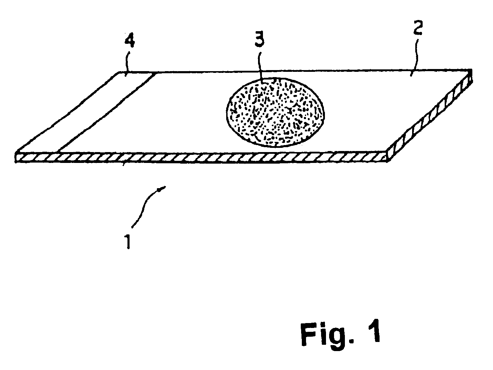

A preparation disk 1 having a filter, used in the present invention will be described with reference to Fig. 1.

A filter 3 has a circular shape, with a diameter of possibly 2cm for instance, matched to the field of sight of an ordinary microscope lens. The filter 3 is located substantially in the center of a disk body 2 except for a frosted part 4, so that it can be readily set on a microscope platform.

The preparation disk may be made of a highly transparent material, such as glass or a plastic. The filter may be obtained by using a laser beam or a charged particle beam. It may be prepared separately and made integral with the disk body by attaching it thereto in alignment with a filter accommodation hole formed in the disk body. Where the filter is initially formed on a plastic preparation disk, holes are formed by using a laser beam. A thinner filter is more desirable from the point of view of mass production. However, the filter should have such a thickness that it is not broken or warped or otherwise distorted by a suction force. The filter 3 has uniformly distributed pores ranging in size from 2.0 to 8.0 microns. For example, when 5-micron cells are to be microscopically observed, a filter with a pore size of from 2 to 5 microns may be used. In this case, cells which are 5 microns or smaller in size are filtered out as waste liquid, while those which are above 5 microns are left on the filter.

The sample preparing apparatus used with the preparation disk having filter according to the invention will now be described. In some embodiments of the invention described below, parts corresponding to those described above are designated by like reference numerals.

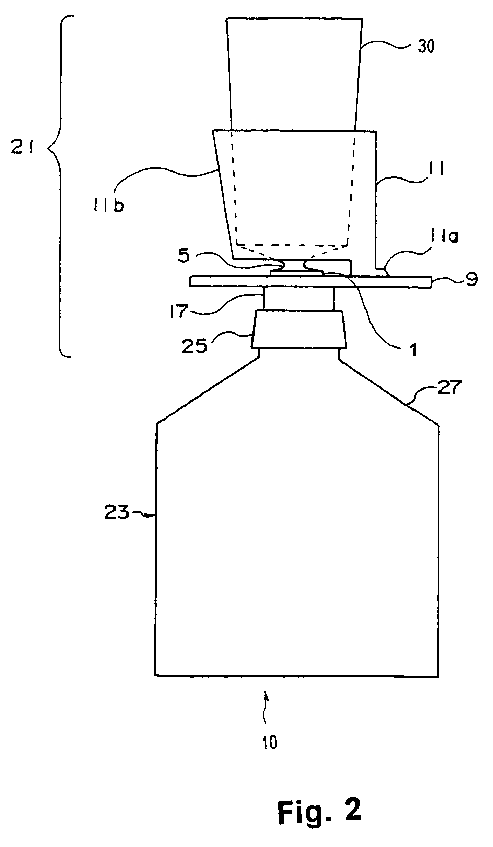

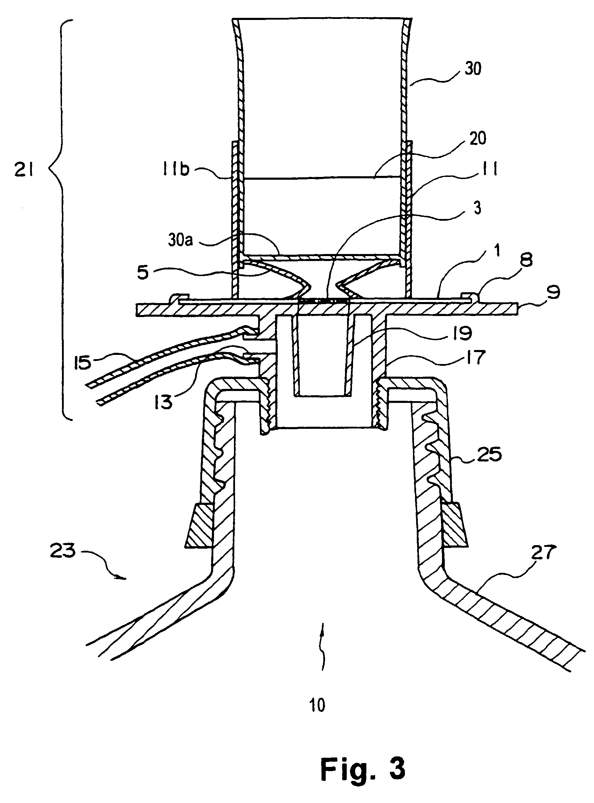

Fig. 2 is a front view showing an embodiment of the sample preparing apparatus used with the preparation disk according to the invention, and Fig. 3 is a fragmentary sectional view showing the same embodiment.

Reference numeral 10 generally designates the sample preparing apparatus used with a preparation disk having a filter. The apparatus comprises a sample preparing section 21 and a discharging section 23. The sample preparing section 21 collects cell from a specimen 20 by filtering the specimen 20 through a filter 3 of a preparation disk 1. The discharging section 23 receives waste liquid filtered out through the filter 3. The sample preparing section 21 is detachably supported on the discharging part 23.

The sample preparing section 21 and discharging section 23 can be tightly and detachably secured to each other by screwing or pressing to facilitate removal. In this embodiment, the two are secured to each other using a cap 25, which is screwed on to a waste liquid container 27 provided on the discharging section side.

A container guide 11 as container guide means is secured to the upper surface of a disk support plate 9 in the sample preparing section 21. The disk support plate 9 has a stopper 8 for holding the preparation disk 1 having a filter. The preparation disk 1 having a filter has a filter 3 integral with substantially the centre of its body. The disk support plate 9 has a hole for discharging the specimen 20 through. The hole is located at a position corresponding to the filter 3 on the preparation disk 1 having a filter supported on the disk support plate 9, and has the same diameter as the filter 3.

The container guide 11 has at least one leg 11a (provided on its rear part in Fig. 3), which is secured by bonding, fusing, or screwing to the disk support plate 9. The container guide 11 has a guide body 11b, which may have a cylindrical shape without a bottom or a shape to clamp the specimen container 30.

A specimen guide 17 for discharging specimen 20 through it is secured on the under side of the disk support plate 9, and it has a suction port 13 formed in its peripheral wall, the suction port 13 being connected to an evacuator. Inside the specimen guide 17, a cylindrical shield 19 is provided to shield the suction port 13. The shield 19 ensures that a specimen 20 passing through the filter 13 in the preparation disk 1 having a filter is discharged through it to the discharging section 23 under the specimen guide without being sucked through the suction port 13.

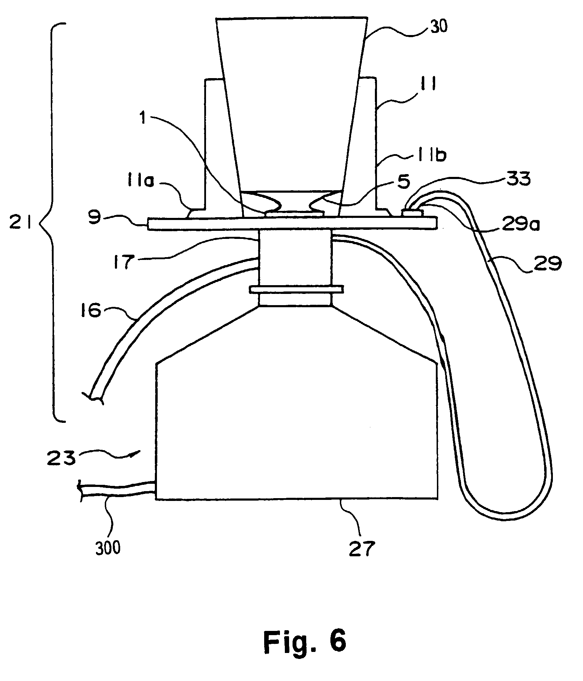

The discharging section 23 receives waste liquid falling as a specimen 20 is filtered, and also detachably supports the sample preparing section 21. In this embodiment, the discharging section 23 includes a waste liquid container 27 for storing waste liquid. It is possible to use a waste liquid container, which has a small volume but still sufficient to store waste liquid for a couple of specimens and has as discharging tube 300 (as shown in Fig. 6) provided near the bottom. The discharging section 23 may not include any waste liquid container. In this case, the discharging section 23 should include a support for supporting the sample preparing section 21 in a stable state.

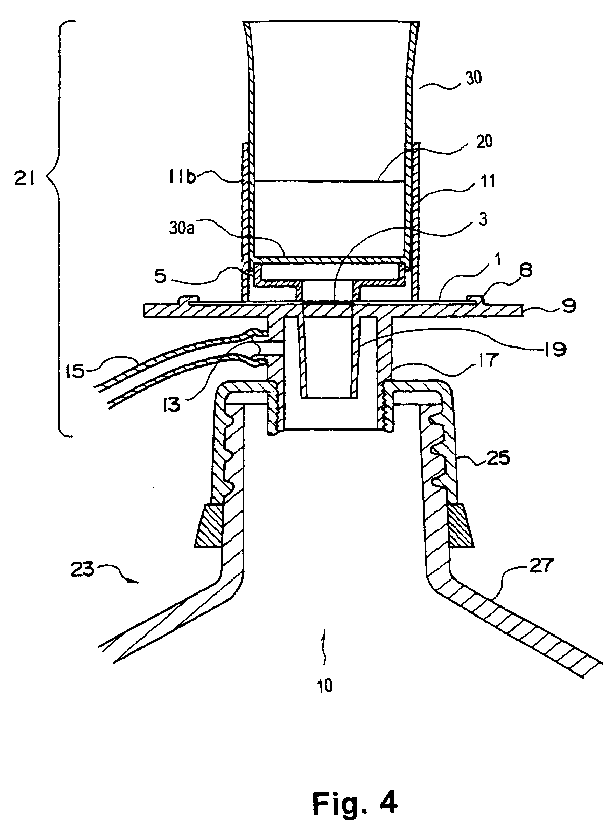

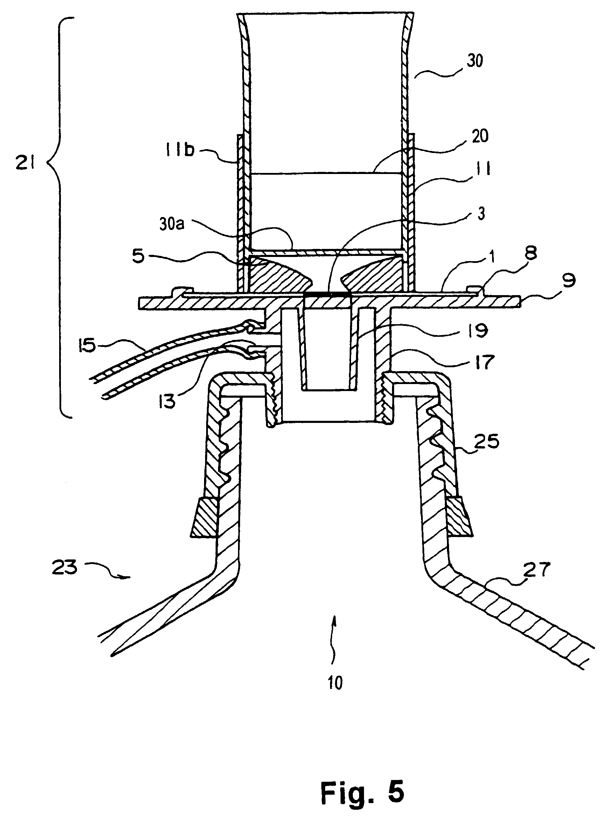

The suction member 5 has a vertical through hole through which specimen can pass and is made of an adequately elastic material, such as rubber or resins. The suction member 5 is located in the body 11b of the container guide 11 at the bottom of the specimen container 30 and can be disposed to air-tightly couple the specimen container 30 and the preparation disk 1. To this end, the suction member 5 has a ring-like shape with one end thereof having the same outer diameter as the specimen container 30 and the other end having the same inner diameter as the outer diameter of the filter 3 of the preparation disk 1 having a filter. Figs. 4 and 5 are fragmentary sectional views similar to Fig. 3 but showing different shape examples of the suction member 5.

The suction member 5 shown in Fig. 4 has two cylindrical parts having large and small diameters. The suction member 5 shown in Fig. 5 has a thick peripheral wall filling a space in the container guide. These suction member examples are basically ring-like in shape like the suction member 5 described above in connection with Figs. 2 and 3. The suction member 5 shown in Fig. 5 is more stable, but its material should be softer.

The method of using the above sample preparing apparatus with the preparation disk having a filter will now be described.

First, the preparation disk 1 having a filter is set on the disk support plate 9. The preparation disk 1 having a filter is held on the disk support plate 9 by the stopper 8, and it is loaded and unloaded by sliding it backwards and forwards.

Next, the suction member 5 is inserted so as to be set under the bottom 30a of the specimen container 30 by pushing it lightly. And then the specimen container 30 with the suction member 5 is set inside the container guide 11 and on the preparation disk 1 having a filter. Depending on the shape of the bottom of the specimen container, the suction member 5 should first be set on the disk support plate 9 and then the specimen container 30 set.

The specimen container 30 may be disposable, and is suitably made of a light and inexpensive material, such as paper or polyethylene. Particularly, when the specimen is urine, and urine extraction paper cup may be used directly.

Subsequently, a switch of the evacuator 15 of the sample preparing apparatus 10 is turned on. As a result, a vacuum valve in the evacuator 15 is operated to evacuate the discharging section 23 (here the waste liquid container 27) to substantially form a vacuum. The specimen container 30, the suction member 5, and the filter 3 of the preparation disk 1 with filter are tightly held in close contact with one another by the suction force thus generated. After confirming the setting of the specimen container 30 and other parts, a hole is formed in the bottom 30a of the specimen container 30 by using an eyelet making tool or similar tool. A specimen 20 is thus sucked from the container 30 and filtered through the filter 3 of the preparation disk 1 having a filter. The waste liquid emerging from the filter 3 flows down through the shield 19 into the discharging section 23. Cells in the specimen thus can be collected on the filter 3. Afterwards, the specimen container 30 and the suction member 5 are taken out from the sample preparing section 21, and the preparation disk 1 having a filter can then be pulled over the disk support plate 9 toward the front in Fig. 3. In this way, collected cells can be obtained as a sample on the preparation disk 1 having filter.

The sequence of operations as described now can be repeated by setting a new preparation disk having a filter, a new suction member, and a new specimen container. In this way, samples of specimens can be successively prepared.

Fig. 6 shows a different embodiment of the sample preparing apparatus according to the invention. This embodiment is the same as the preceding embodiment except that a bypass duct 29 is provided. The bypass duct 29 is tubular in shape and extends from the peripheral wall of the specimen guide 17, with an end inserted in a recess portion 33 provided on the upper surface of the disk support plate 9. It is in communication with the space in the waste liquid container 27, and is thus held in a vacuum when the sample preparing apparatus 10 is used.

In the use of the sample preparing apparatus 10, specimen 20 may remain in the specimen container 30 after being sucked. For example, when extraordinary cells such as cancer cells are contained in a specimen 20, they are greater in number than the number of ordinary cells, and a liquid residue may result from clogging of the filter 3. In such a case, the end 29a of the bypass duct 29 is taken out from the recessed portion 33 on the disk support plate 9 and put into the liquid residue in the specimen container 30. As described above, the bypass duct 29 is in communication with the waste liquid container 27, which is substantially in vacuum. The liquid residue can thus be discharged through the bypass duct 29 into the waste liquid container 27.

In the illustrated specimen preparing apparatus 10, the waste container 27 which constitutes the discharging section 23 has a small volume sufficient for storing waste liquid for only a couple of specimens and is provided with a discharging duct 300. The apparatus thus uses an evacuator 16, in which by turning a switch (not shown) on, an evacuating valve is opened to start suction from an internal pump, and by turning the switch off, a discharging valve causes waste liquid that has been tentatively stored in the in the waste liquid container 27 to be discharged by the pump through the discharging duct 300. It is thus possible to collect cells easily and efficiently by discharging waste liquid for every couple of specimens by repeatedly on-off operating of the switch.

The preparation disk having a filter with collected cells thereon is immediately fixed by a rapid spray process or the like, and then treated using ordinary Papanicolaou staining or the like. The Papanicolaou staining is used with an extender introduced in a layer immediately before the last xylole layer. When the filter of the preparation disk having a filter is made of polycarbonate, it may be wrinkled by dipping it in an alcohol bath. The extender has the effect of extending the filter and thus facilitating focusing. After the staining treatment, the preparation disk having a filter is sealed with cover glass by attaching a sealing agent, for instance marinol, to it, and a de-poring sheet is fitted with a small amount of marinol or the like attached to it on the rear side of the filter, thus completing the sample preparation.

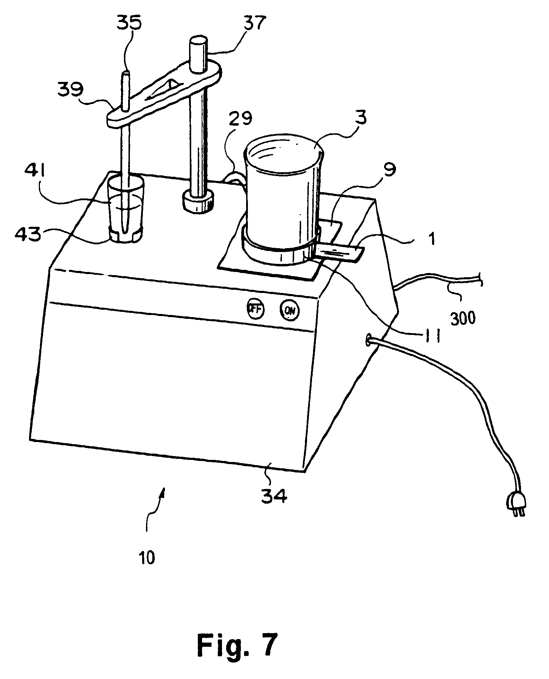

Fig. 7 is a perspective view showing another embodiment of the sample preparing apparatus according to the invention.

A portion of the sample preparing section 21 below the disk support plate 9 and a portion of the discharging section 23 are accommodated in a box-like frame 34, while only a portion for manual operation is exposed to the outside. The disk support plate 9 occupies a portion of the upper surface of the frame 34. A specimen 20 is filtered through the filter 3 of the preparation disk 1 having a filter to collect cells in the manner as described. In this embodiment, an awl 35 for forming a hole in the bottom of the specimen container 30 is provided above the frame 34. The awl 35 is secured to a projection 39 projecting perpendicularly from the upper end of a vertically slidable shaft 37. A washing vessel 41 for washing the awl 35 is supported on a vessel guide 43. When the awl 35 is not being used, its lower end portion is held dipped in the washing vessel 41. When using the awl 35, a power switch (not shown) is turned on to set the specimen container 30 in the container guide 31 as described. Then, the awl 35 in the washer vessel 41 is raised by raising the shaft 37, then moved horizontally to a position above the specimen container 30, and then lowered to push and open a hole in the bottom of the specimen container 30. The lower limit position of the awl 35, where the awl 35 reaches its bottom end at the lowermost position, is set such that it is several millimetres above the filter 3 of the preparation disk 1 having a filter. The awl 35 is adapted to be angularly moved horizontally between a position corresponding to the center of the washing vessel 41 and a position corresponding to the center of the specimen container 30. The awl 35 can thus readily open a hole in the center of the container and can also be readily inserted into the washing vessel 41. It is thus possible to form a hole with the awl 35 without controlling the force applied thereto and also without the need to position the awl 35 for forming the hole thus permitting easy treatment of successive specimens. The washing vessel 41 can be removed from the vessel guide 43 for replacement when necessary.

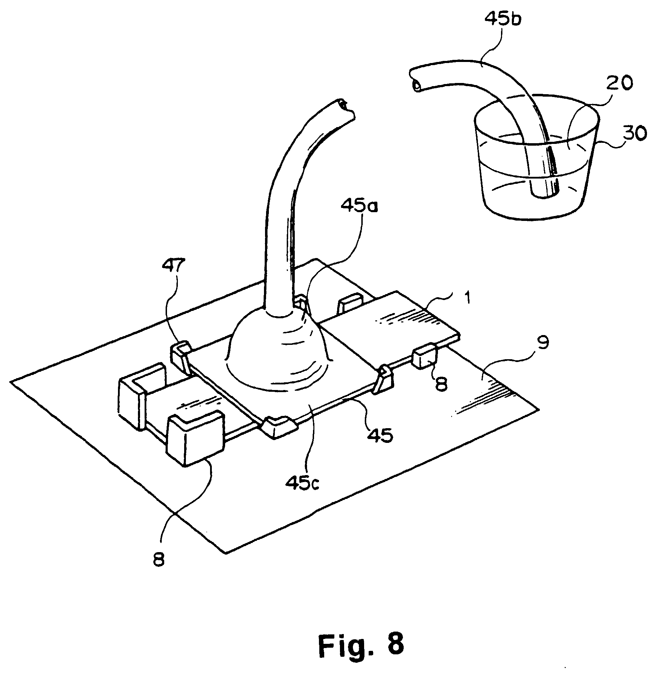

Fig. 8 is a perspective view showing a further embodiment of the sample preparing apparatus according to the invention which uses a bell-shaped suction member 45. The bell-shaped suction member 45 has a bell-shaped large-diameter portion 45a having substantially the same inner diameter as the diameter of the filter 3 of the preparation disk 1 with filter. The suction member 45 also has a pipe-like sucking portion 45b, and a rectangular lower end portion 45c which is greater in size than the short side of the preparation disk 1 having a filter. A suction member support means for supporting the suction member 45 is provided on the disk support plate 9. L-shaped stoppers 47 as the suction support means for retaining the four corners of the lower end portion 45c of the suction member are provided on the disk support plate. In use, after setting the preparation disk 1 having a filter on the disk support plate 9, the suction member 45 is secured over the filter 3 by hooking its lower end portion 45c in the stoppers 47. Subsequently, the switch of the sample preparing apparatus is turned on and then the end of the pipe-like sucking portion 45b of the sucking member 45 is inserted into the specimen container 30 for filtering the specimen 20 and collecting cells thereof. The suction member 45 is desirably disposable. In this embodiment, it is not necessary to form any hole in the specimen container bottom, so the operation is further simplified.

## Claims

1. A sample preparing apparatus (10) used with a preparation disk (1) having a preparation disk body (2) and an integral filter (3), the apparatus (10) comprising:
a sample preparing section (21), which includes a disk support plate (9) having a holder (8) for releasably holding said prepartion disk (1) on the upper surface of the disk support plate (9), and a specimen guide (17) for discharging a specimen (20) passed through the filter (3) of said preparation disk (1) which is secured to the underside of said disk support plate (9) and which has a suction port (13) connected to an evacuator;
a suction member (5; 45) to couple a specimen container (30) containing the specimen (20) therein with the filter (3) of said preparation disk (1) and a discharging section (23) coupled to said sample preparing section (21).

2. The sample preparing apparatus (10) according to claim 1, wherein said suction member (5) has one end having the same inner diameter as the outer diameter of the filter (3) of said preparation disk (1) and the other end having the same outer diameter as the outer diameter of the bottom of said specimen container (30).

3. The sample preparing apparatus (10) according to claim 1, wherein said suction member (45) has a large-diameter portion (45a) with one end thereof having substantially the same inner diameter as the diameter of the filter (3) of said preparation disk (1), and a pipe-like suction portion (45b) extending from the other end of the large-diameter portion (45a).

4. The sample preparing apparatus (10) according to claim 2, wherein:
said sample preparing section (21) further includes container guide means (11) secured to the upper surface of said support plate (9) and supporting said suction member (5) and said specimen container (30) above said preparation disk (1).

5. The sample preparing apparatus (10) according to claim 3, wherein:
said sample preparing section (21) further includes suction member support means (47) secured to said disk support plate (9) for supporting said suction member (45) on the upper surface of said prepartion disk (1).

6. The sample preparing apparatus (10) according to claim 4 or 5, wherein said sample preparing section (21) further includes a shield (19) disposed inside said specimen guide (17) which shields specimen (20) falling through said specimen guide (17) from said suction port (13).

7. The sample preparing apparatus (10) according to claim 4, further comprising a bypass duct (29) extending from a peripheral wall of said specimen guide (17) and used for processing a liquid residue.
